**Europäisches Patentamt**

(19) **European Patent Office**

**Office européen des brevets**

(11) Veröffentlichungsnummer: **0 006 577**
**B1**

(12) **EUROPÄISCHE PATENTSCHRIFT**

(45) Veröffentlichungstag der Patentschrift:
**25.11.81**

(51) Int. Cl.³: **A 61 B 5/00,** A 61 M 25/00,
G 01 L 9/00

(21) Anmeldenummer: **79102045.6**

(22) Anmeldetag: **20.06.79**

(54) **Katheter zum Einführen in ein Gefäss eines Patienten.**

(30) Priorität: **27.06.78 DE 2828206**

(43) Veröffentlichungstag der Anmeldung:
**09.01.80 Patentblatt 80/1**

(45) Bekanntmachung des Hinweises auf die Patenterteilung:
**25.11.81 Patentblatt 81/47**

(84) Benannte Vertragsstaaten:
**FR GB NL SE**

(56) Entgegenhaltungen:
**AT-B-282 810**
**DE-A-2 117 541**
**DE-A-2 150 378**
**DE-A-2 346 270**
**DE-A1-2 447 529**
**DE-B-1 262 503**
**US-A-3 437 088**

(73) Patentinhaber: **SIEMENS AKTIENGESELLSCHAFT Berlin und München, Postfach 22 02 61, D-8000 München 22 (DE)**

(72) Erfinder: **Lindström, Kjell, Dr., Skogsvägen 3, S-23012 Höllviksnäs (SE)**
Erfinder: **Ulmsten, Ulf, Dr.med., Geijersgatan 39, S-21618 Malmö (SE)**
Erfinder: **Hök, Bertil, Dr., Noréensväg 70, S-75263 Uppsala (SE)**
Erfinder: **Nilsson, Kenth, Trälhavsvägen 42, S-18400 Akersberga (SE)**

Katheder zum Einführen in ein Gefäß eines Patienten

Die Erfindung betrifft einen Katheter zum Einführen in ein Gefäß eines Patienten, der über seine Länge verteilt mindestens zwei Druckwandler zum Messen des Flüssigkeitsdruckes im Gefäß an verschiedenen Stellen aufweist.

Bei vielen klinischen Untersuchungen ist es für eine korrekte Diagnostik von Bedeutung, gleichzeitige Druckmessungen an mehreren Meßpunkten ausführen zu können. Ein Beispiel ist die Messung von Drücken auf beiden Seiten einer Herzklappe sowohl bei einer Systole als auch bei einer Diastole, wobei Stenosen und in gewissen Fällen Insuffizienzen entdeckt werden können. Auch andere Organe, die peristaltische Bewegungen ausführen, z. B. Ösophagus, Uterus, werden häufig mittels manometrischer Technik untersucht. So kann bei der Untersuchung der unteren Urinwege eine normale Funktion und eine Disfunktion am besten durch gleichzeitige Druckmessung in der Blase und in der Urethra festgestellt werden.

Bei solchen Druckmessungen werden häufig flüssigkeitsgefüllte Katheter verwendet, die an externen Druckmessern angeschlossen werden. Aufgrund der verhältnismäßig langen Übertragungswege der Flüssigkeitsdrücke können dabei aber die Meßwerte verfälscht werden. Ein Katheter der eingangs genannten Art ist durch die Zeitschrift »Acta Obstet Gynecol Scaud« 55, Seiten 167 bis 173, 1976 bekannt. Die auf dem Katheter angebrachten Miniaturdruckwandler sind elektrische Dehnungselemente aus Silizium. Da diese Druckwandler aufgrund ihrer Bruchempfindlichkeit verhältnismäßig groß gehalten werden müssen, können sie in einem Katheter nicht dichter als in einem Abstand von ca. 20 mm zueinander angeordnet werden. Werden mehr als drei Druckwandler auf einem Katheter normaler Länge angebracht, so wird er sehr steif und ist deshalb nicht mehr geeignet, in ein biologisches Gewebe eingeführt zu werden. Da es z. B. bei der Messung der Drücke in der Urethra erwünscht wird, an mehreren einander naheliegenden Stellen zu messen, damit eine Profilkurve der Wanddrücke enthalten wird, ist der bekannte Katheter an einer Anordnung zum kontinuierlichen Ziehen des Katheters angeschlossen, so daß an mehreren Stellen gemessen werden kann. Beim Ziehen des Katheters entstehen aber leicht Bewegungsartefakte, die zu falschen Meßwerten führen. Ferner ist die Anordnung zum Ziehen des Katheters sperrig und teuer.

Der Erfindung liegt die Aufgabe zugrunde, einen Katheter der eingangs genannten Art zu schaffen, bei dem mehr Druckwandler als bisher mit kleineren Abständen angebracht werden können.

Diese Aufgabe ist erfindungsgemäß dadurch gelöst, daß jeder Druckwandler eine Meßzelle aufweist, die an einem Ende geschlossen ist, mit ihrem offenen Ende einer Öffnung in der Wand des Katheters gegenüberliegt und in deren Innenraum zwei Elektroden zur Messung des Widerstandes der in die Meßzelle hineingedrückten Flüssigkeitssäule ragen. Aufgrund der Tatsache, daß der Druckwandler aufgrund seines stabilen Aufbaus kleiner als die bisherigen Druckwandler gehalten werden kann, können nunmehr mehr Druckwandler als bisher mit kleinen Abständen voneinander an dem Katheter angebracht werden.

In einer vorteilhaften Ausbildung der Erfindung wird vorgeschlagen, daß der Katheter aus mehreren, parallel geführten schlauchartigen Katheterelementen besteht, von denen jedes mindestens einen Druckwandler aufweist. Dadurch ist erreicht, daß der Katheter beliebig viele, beliebig nahe beieinander liegende Druckmeßpunkte beinhalten kann, so daß eine Profilkurve registriert werden kann, ohne den Katheter während des Messens bewegen zu müssen.

In den meisten rohrförmigen Körperhöhlen, z. B. Ösophagus, Uterus und Urethra, ist eine gut entwickelte Schleimhaut vorhanden, die im Ruhezustand das innere Lumen der entsprechenden Körperhöhle begrenzt. Die Schleimhaut deckt deshalb leicht die Meßstellen eines eingeführten Katheters. Um dies zu vermeiden, ist es bekannt, Flüssigkeit einzuführen, die aber die Schleimhaut beeinflussen kann, ohne mit Sicherheit die Meßartefakte zu eliminieren.

Davon ausgegangen wird in einer Weiterbildung der Erfindung vorgeschlagen, daß jede Öffnung eines Katheterelements zur Symmetrieachse des Katheters gerichtet ist und daß die parallel geführten Katheterelemente im Bereich einer Öffnung einen Abstand voneinander aufweisen, der mit Flüssigkeit füllbar ist. Dadurch ist erreicht, daß die Schleimhaut die Druckmeßstellen des Katheters nicht erreicht.

In einer weiteren Ausbildung der Erfindung wird vorgeschlagen, daß die Meßzelle in einem Formstück eingebettet ist, das eine seitliche Öffnung aufweist, an seinen beiden Enden geschlossen und dort zum Anschluß an den Katheter ausgebildet ist. Dadurch ist erreicht, daß ein Modulaufbau eines Katheterelements möglich ist. Aufgrund des Modulaufbaus können die wechselnden Forderungen, die im Zusammenhang mit den verschiedenen Applikationen an die Anzahl der Meßpunkte der Katheter gestellt werden, leicht erfüllt werden. Es kann also eine Mehrzahl von Typen von Kathetern geschaffen werden, bei denen jeder einzelne an eine aktuelle Meßsituation angepaßt ist.

Die Erfindung ist nachfolgend anhand eines in den Zeichnungen dargestellten Ausführungsbeispiels näher erläutert.

Es zeigt

Fig. 1 ein Katheterelement nach der Erfindung,

Fig. 2 einen Schnitt durch das Katheterelement nach Fig. 1 nach der Linie II-II,

Fig. 3 eine Seitenansicht eines Katheters, bestehend aus drei Katheterelementen nach Fig. 1 und 2,

Fig. 4 bis 7 Querschnitte durch den Katheter nach Fig. 3,

Fig. 8 eine schematische Darstellung einer Ausführungsform eines Katheters nach den Fig. 1 bis 7,

Fig. 9 eine weitere schematische Darstellung einer Ausführungsform eines Katheters nach den Fig. 1 bis 7 und

Fig. 10 einen Querschnitt durch den Katheter in Fig. 9 nach der Linie X-X.

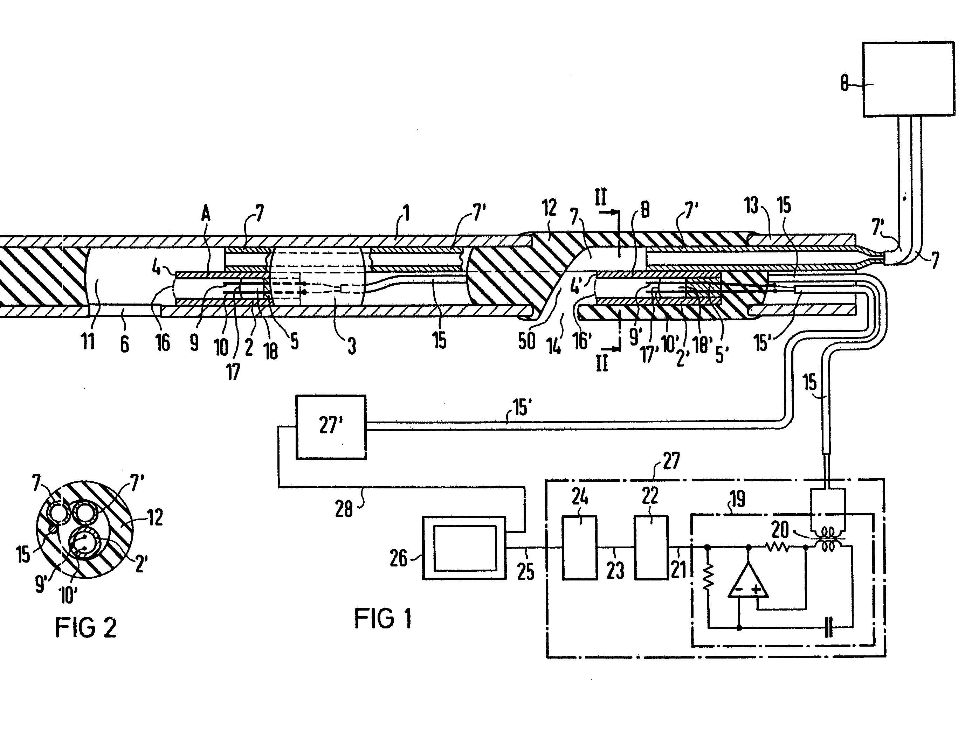

Die Fig. 1 zeigt, daß ein Katheterelement ein biegsames Rohr 1 aufweist, in den ein Miniaturdruckwandler A angeordnet ist. Der Druckwandler A weist eine Meßzelle 2 auf, die exzentrisch im Rohr 1 angeordnet und mittels einer Füllmasse 3, z. B. aus Silikongummi, befestigt ist. Die Meßzelle 2 ist an ihrem dem Meßende 4 des Druckwandlers A abgewandten Ende 5 geschlossen. Der Druckwandler ist ferner über eine Öffnung 6 im Rohr 1 mit der Umgebung des Katheterelements verbunden. Die Meßzelle 2 endet im Abstand zur Öffnung 6 des Rohres 1 und ist dort offen. Das freie Ende des Rohres 1 ist mit einer Füllmasse, z. B. Silikon, gefüllt, die auch als Wand für den Raum 11 vor dem Druckwandler dient. Zwischen dem Rohr 1 und der Meßzelle 2 ist ein Spalt für eine Flüssigkeitsleitung 7 freigelassen, die an einer Flüssigkeitspumpe 8 mit Kochsalzlösung angeschlossen ist. Die Flüssigkeitspumpe 8 kann ein Behälter sein, in dem Kochsalzlösung unter einem bestimmten Druck steht. Der Flüssigkeitswiderstand und damit der Durchmesser der Flüssigkeitsleitung 7 ist an den gewünschten Flüssigkeitsfluß angepaßt. In der Meßzelle 2 sind zwei Elektroden 9, 10 aus Platin im Abstand in Rohrlängsrichtung angeordnet, die an einer später näher beschriebenen Schaltungsanordnung zur Messung des jeweiligen Widerstandes zwischen ihnen angeschlossen sind.

Das Katheterelement kann beliebig verlängert werden, indem Formstücke, z. B. aus Silikongummi, mehrere Rohre miteinander verbinden. So verbindet in der Fig. 1 das Formstück 12 die Rohre 1 und 13. In jedem Formstück ist ein Druckwandler eingebettet, der über eine Öffnung mit der Umgebung des Katheterelementes in Verbindung steht. In dem Formstück 12 ist ein Druckwandler B eingebettet, der die gleichen Bezugszahlen hat wie der Druckwandler A. Zur Unterscheidung ist aber ein Strich beigefügt. Der Raum vor dem Druckwandler B ist mit 50 bezeichnet.

Fig. 2 zeigt, daß die Flüssigkeitsleitung 7 des Druckwandlers A, die mit der Flüssigkeitspumpe 8 in Verbindung steht, das Formstück 12 durchsetzt. Die Leitung 15, die die Elektroden 9, 10 mit der später beschriebenen Schaltungsanordnung verbindet, ist auch durch das Formstück 12 geführt.

Wenn eine Druckmessung vorgenommen werden soll, müssen die Druckwandler mit Kochsalzlösung gefüllt werden, bevor sie direkt am Meßbereich appliziert werden. Im folgenden wird der Meßvorgang für den Druckwandler A beschrieben. Das gleiche gilt auch für den Druckwandler B. Es strömt Kochsalzlösung von der Flüssigkeitspumpe 8 über die Flüssigkeitsleitung 7 in den Raum 11 hinein und drückt die im Druckwandler A vorhandene Luft hinaus. Aufgrund der kleinen Dimensionen des Druckwandlers strömt die Kochsalzlösung, abhängig vom Druck der Flüssigkeitspumpe, nicht in die Umgebung hinaus, sondern füllt den Raum 11 im Rohr 1. Aufgrund der Ausbildung und Lage der Meßzelle 2 wird die darin befindliche Luft zunächst nicht entfernt. Es bildet sich genau an dem freien Ende der Meßzelle 2 ein Meniskus 16, wie es die strichpunktierte Linie zeigt.

Um eine Druckmessung vornehmen zu können, muß der Meniskus 16, d. h. die Kochsalzlösung, in die Meßzelle 2 hineingeführt werden, so daß die freien Enden der Elektroden 9, 10 in die Kochsalzlösung eintauchen. Der Meniskus 16 muß eine definierte Lage in der Meßzelle 2 haben. In der Fig. 1 ist der in die Meßzelle 2 geschobene Meniskus mit 17 bezeichnet.

Damit eine genaue Festlegung der Lage des Meniskus möglich ist, kann, bevor der Druckwandler mit Kochsalzlösung gefüllt wird, die Meßzelle 2 mit einer Gasmischung aus $CO_2$ und Luft gefüllt werden. Wenn danach der Druckwandler A mit Kochsalzlösung gefüllt wird, wird sie aufgrund der Auflösung des $CO_2$ dieser Gasmischung in der Kochsalzlösung in die Meßzelle 2 hineingesaugt, und zwar bis zu einem Niveau, das von der Zusammensetzung der Gasmischung abhängt. Durch diese Zusammensetzung kann also die Lage des Meniskus in der Meßzelle 2 festgelegt werden.

Das Katheterelement wird für eine Druckmessung, z. B. in ein Gefäß eines Patienten eingeführt, wobei sich das in der Meßzelle 2 eingeschlossene Gasvolumen 18 in Abhängigkeit vom Druck im Gefäß ändert. Das jeweilige Gasvolumen 18 entspricht dem Druck der Kochsalzlösung und damit dem zu messenden Druck und wird in ein elektrisches Signal über die Elektroden 9, 10 umgewandelt.

Der Widerstand zwischen den Elektroden 9, 10 ist von der Lage des Meniskus 17, der das Luftvolumen 18 begrenzt, abhängig. Dieser Widerstand beeinflußt die Frequenz eines Oszillators 19 einer Schaltungsanordnung 27. Beim Verbinden des Druckwandlers A bzw. der Elektroden 9, 10 mit der Sekundärwicklung eines Transformators 20 des Oszillators 19 wird ein vollständige galvanische Isolierung zwischen dem Druckwandler A und dem Oszillator 19 erhalten, was aus Sicherheitsgründen wünschenswert ist. Ein Oszillator dieser Art ist z. B. in der Literaturstelle »Operational Amplifiers«, Seite 370, McGraw-Hill, New York, 1971, beschrieben. Der Oszillator 19 ist über die Verbindung 21 mit einem Gerät 22 zum Umwandeln der Frequenz in eine entsprechende Spannung verbunden. Das Gerät 22 ist seinerseits über die Verbindung 23 mit einem Verstärker 24 zum Verstärken der Signale vom Gerät 22 verbunden. Der Verstärker 24 ist wiederum über die Verbindung 25 mit einem Aufzeichnungsgerät 26 zum Aufzeichnen der Meßergebnisse verbunden. Jeder in dem Katheterelement vorhandene Druckwandler ist mit einer solchen Schaltungsanordnung verbunden. Der Druckwandler B ist demgemäß über die Verbindung 15' an einer weiteren Schaltungsanordnung 27' dieser Art angeschlossen, die über die Verbindungsleitung 28 ebenfalls mit dem Aufzeichnungsgerät 26 verbunden ist.

Die Dimensionen eines Druckwandlers können sehr klein gehalten werden. Bei einem Ausführungsbeispiel war der äußere Durchmesser 0,9 mm einschließlich des Rohres 1 und die Länge 4 mm.

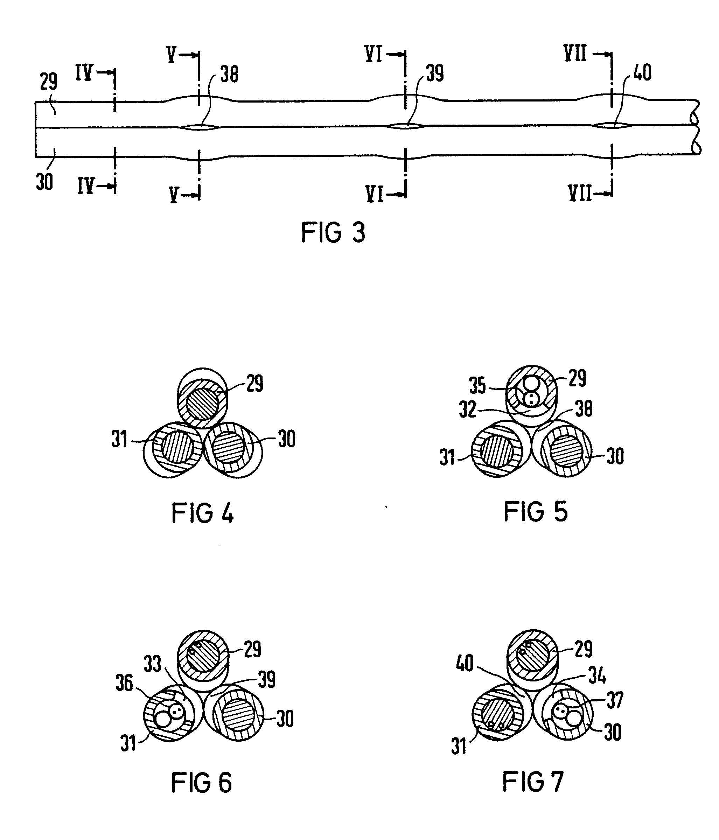

In Fig. 3 besteht der Katheter aus drei parallel geführten, gebündelten Katheterelementen 29, 30, 31. In den Fig. 4 bis 7 ist zu sehen, daß er im Querschnitt dreiecksförmig aufgebaut ist. In den Fig. 3 und 4 bis 7 ist auch ersichtlich, daß jede Öffnung 32, 33, 34 eines Katheterelements 29, 30, 31 an dem jeweiligen Druckwandler 35, 36, 37 zur Symmetrieachse des Katheters hin gerichtet ist. die Katheterelemente 29, 30, 31 weisen ferner im Bereich der Öffnungen 32, 33, 34 einen Abstand voneinander auf, so daß ein Hohlraum 38, 39, 40 gebildet ist, der mit Flüssigkeit als Druckübertragungsmittel füllbar ist. Bei einer Druckmessung in einer Körperhöhle sind diese Hohlräume 38, 39, 40 mit Kochsalzlösung gefüllt, wobei der zu messende Druck über diese Kochsalzlösung übertragen wird. Aus den Fig. 3 bis 7 geht hervor, daß der Druckwandler 35 an der Stelle V, der Druckwandler 36 an der Stelle VI und der Druckwandler 37 an der Stelle VII angeordnet sind. Die Druckwandler 35 bis 37 messen also an über die Länge des Katheters versetzten Stellen den Druck. Es ist also möglich, bei dem Beispiel gemäß den Fig. 3 bis 7 an drei verschiedenen Stellen den Druck in einem Gefäß eines Patienten gleichzeitig zu messen. Die Druckwandler 35 bis 37 können dabei in einem relativ geringen Abstand zueinander angeordnet sein. Aufgrund der Tatsache, daß die evtl. vorhandene Schleimhaut die Öffnungen 32, 33, 34 nicht abdecken kann, weil diese nach innen gerichtet sind, ist eine Verfälschung der Meßwerte vermieden.

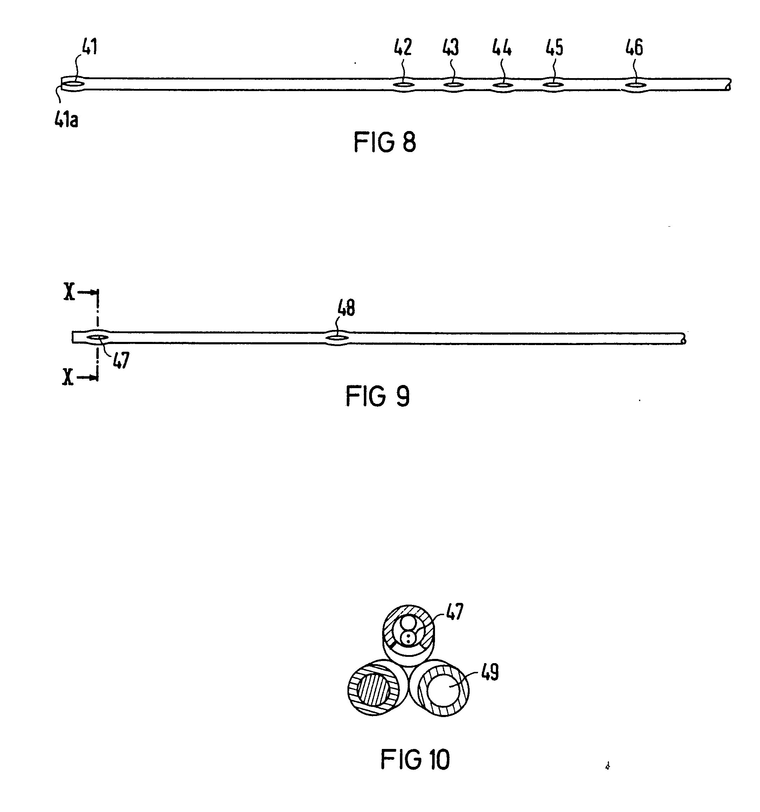

Die Fig. 8 zeigt schematisch den Aufbau eines Katheters mit drei parallel geführten, im Querschnitt dreiecksförmig angeordneten Katheterelementen gemäß den Fig. 3 bis 7. Dieser Katheter ist für die Messung des Druckes in der Urethra geeignet. An dem Katheter sind sechs Meßpunkte, d. h. sechs Druckwandler 41 bis 46, angeordnet. Der Aufbau und die Funktion dieser Druckwandler ist bereits im Zusammenhang mit den Fig. 1 bis 7 beschrieben. Der Abstand zwischen den Druckwandlern 41 und 42 ist ca. 40 mm, zwischen den Druckwandlern 42 und 43, 43 und 44, 44 und 45 beträgt er ca. 6 mm und der Abstand zwischen den Druckwandlern 45 und 46 beträgt ca. 10 mm. Das distale Ende 41a des Katheters wird durch die Urethra geschoben und so appliziert, daß der Druckwandler 41 den Druck in der Blase und die Druckwandler 42 bis 46 den Druck in der Urethra messen können.

Die Fig. 9 zeigt ein weiteres Ausführungsbeispiel eines Katheteraufbaus, bei dem zwei Druckwandler 47, 48 vorhanden sind. Bei Druckmessungen, bei denen zwei Meßpunkte ausreichen, z. B. wenn auf jeder Seite einer Herzklappe gemessen werden soll, kann ein drittes Katheterelement ohne Druckwandler z. B. zur Abnahme von Blutproben oder für Injektion von Röntgenkontrastmittel und Farmaka, also als Flüssigkeitsleitung, dienen.

Fig. 10 zeigt, daß der Kanal 49 des einen Katheterelements frei z. B. für die Abnahme von Blutproben ist.

Die Verbindung der Katheterelemente kann durch Leimen, Schweißen oder Festklemmen mit Hilfe von speziellen Befestigungselementen, die die Katheterelemente an bestimmten Punkten festhalten, erfolgen. Bei der zuletzt genannten Ausführung ist es möglich, die Katheterelemente und die Befestigungselemente als Bausatz zu liefern, wobei der Benutzer ein System nach eigenen Wünschen aufbauen kann.

## Patentansprüche

1. Katheter zum Einführen in ein Gefäß eines Patienten, der über seine Länge verteilt mindestens zwei Druckwandler (A, B, 35, 36, 37, 41 bis 48) zum Messen des Flüssigkeitsdruckes im Gefäß an verschiedenen Stellen aufweist, dadurch gekennzeichnet, daß jeder Druckwandler (A, B, 35, 36, 37, 41 bis 48) eine Meßzelle (2, 2') aufweist, die an einem Ende (5, 5') geschlossen ist, mit ihrem offenen Ende (4, 4') einer Öffnung (6, 14, 32, 33, 34) in der Wand des Katheders gegenüberliegt und in deren Innenraum zwei Elektroden (9, 10, 9', 10') zur Messung des Widerstandes der in die Meßzelle (2, 2') hineingedrückten Flüssigkeitssäule ragen.

2. Katheter nach Anspruch 1, dadurch gekennzeichnet, daß er aus mehreren, parallel geführten schlauchartigen Katheterelementen (29, 30, 31) besteht, in denen die Druckwandler (35, 36, 37, 41 bis 48) angeordnet sind.

3. Katheter nach Anspruch 2, dadurch gekennzeichnet, daß die Katheterelemente (29, 30, 31) vorzugsweise im Querschnitt dreiecksförmig gebündelt sind, daß jede Öffnung (32, 33, 34) eines Katheterelementes zur Symmetrieachse des Katheters gerichtet ist und daß die parallel geführten Katheterelemente (29, 30, 31) im Bereich einer Öffnung einen Abstand voneinander aufweisen, der einen Hohlraum (38, 39, 40) bildet, der mit Flüssigkeit als Druckübertragungsmittel füllbar ist.

4. Katheter nach Anspruch 1 bis 3, dadurch gekennzeichnet, daß die Meßzelle (2') in einem Formstück (12) eingebettet ist, das eine seitliche Öffnung (14) aufweist, an seinen beiden Enden geschlossen und dort zum Anschluß an den Katheter ausgebildet ist.

5. Katheter nach einem der Ansprüche 2 bis 4, dadurch gekennzeichnet, daß in mindestens einem Katheterelement (29, 30, 31) kein Druckwandler angeordnet ist, so daß es als Flüssigkeitsleitung benutzbar ist.

## Claims

1. A catheter for inserting into a channel of a patient, possessing at least two pressure converters (A, B, 35, 36, 37, 41 to 48) distributed over its length which serve to measure the liquid pressure in a channel at different points, characterised in that each pressure converter (A, B, 35, 36, 37, 41 to 48) has a test cell (2, 2') which is sealed at one end (5, 5') and whose open end (4, 4') is opposite an aperture (6, 14, 32, 33, 34) in the side of the catheter and into whose interior space project two electrodes which serve to measure the resistance of the liquid column pushed into the test cell (2, 2').

2. A catheter as claimed in claim 1, characterised in that it consists of a plurality of parallel directed, hose-like catheter elements (29, 30, 31) in which the pressure converters (35, 36, 37, 41 to 48) are arranged.

3. A catheter as claimed in claim 1, characterised in that the catheter elements (29, 30, 31) are preferably triangular bundles in cross-section, that each aperture (32, 33, 34) of a catheter element is directed towards the axis of symmetry of the catheter, and that in the region of an aperture, the parallel directed catheter elements (29, 30, 31) have a mutual spacing which forms a hollow space (38, 39, 40) which can be filled with liquid as pressure transfer means.

4. A catheter as claimed in claims 1 to 3, characterised in that the test cell (2) is embedded in a shaped part (12) which has a lateral opening (14), is sealed at its two ends and designed to serve as a connection to the catheter.

5. A catheter as claimed in one of claims 2 to 4, characterised in that at least one catheter element (29, 30, 31) does not contain any pressure converter, so that the element can be used as liquid pipe.

## Revendications

1. Cathéter destiné à être introduit dans un vaisseau d'un malade, qui présente au moins deux capteurs (A, B, 35, 36, 37, 41 à 48) de pression répartis sur la longueur pour mesurer la pression du liquide dans le vaisseau en des endroits différents, caractérisé en ce que chaque capteur (A, B, 35, 36, 37, 41 à 48) de pression comprend une cellule (2, 2') de mesure, qui est fermée à l'une des extrémités (5, 5'), dont l'extrémité (4, 4') ouverte est en face d'une ouverture (6, 14, 32, 33, 34) ménagée dans la paroi du cathéter, et à l'intérieur de laquelle font saillie deux électrodes (9, 10, 9', 10') destinées à mesurer la résistance de la colonne de liquide poussée dans la cellule (2, 2') de mesure.

2. Cathéter suivant la revendication 1, caractérisé en ce qu'il est constitué de plusieurs éléments (29, 30, 31) de cathéter parallèles en forme de tube souple, dans lesquels sont disposés les capteurs (35, 36, 37, 41 à 48) de pression.

3. Cathéter suivant la revendication 2, caractérisé en ce que les éléments (29, 30, 31) de cathéter se présentent sous la forme d'un faisceau, de préférence de section droite triangulaire, chaque ouverture (32, 33, 34) d'un élément de cathéter est dirigé vers l'axe de symétrie du cathéter et les éléments (29, 30, 31) parallèles du cathéter sont dans la région d'une ouverture à un intervalle les uns des autres qui forme un espace (38, 39, 40) vide qui peut être empli du liquide servant de moyen de transmission de la pression.

4. Cathéter suivant la revendication 1 à 3, caractérisé en ce que la cellule (2') de mesure est noyée dans une pièce (12) moulée, qui présente une ouverture (14) latérale, qui est fermée aux deux extrémités et qui y est agencée de manière à se raccorder au cathéter.

5. Cathéter suivant l'une des revendications 2 à 4, caractérise en ce qu'il n'y a pas de capteur de pression dans au moins un élément (29, 30, 31) de cathéter de sorte qu'il peut être utilisé comme conduit pour du liquide.

FIG 1

FIG 2

FIG 3

FIG 4

FIG 5

FIG 6

FIG 7

FIG 8

FIG 9

FIG 10